# EUROPEAN PATENT SPECIFICATION

(11) **EP 3 458 075 B1**
(45) Date of publication and mention of the grant of the patent: **26.04.2023**
(21) Application number: 17725797.9
(22) Date of filing: 18.05.2017
(51) Int. Cl.: A61K 33/40, A61K 33/14, A61P 17/10, A61P 17/02, A61K 9/00

(54) **NEW USE OF PRODUCT FOR SKIN TREATMENT**
NEUE VERWENDUNG VON PRODUKTEN ZUR HAUTBEHANDLUNG
NOUVELLE UTILISATION D'UN PRODUIT POUR LE TRAITEMENT DE LA PEAU

(30) Priority: 19.05.2016 SE 1650679
(43) Date of publication of application: 27.03.2019
(73) Proprietor: Biocool AB, 102 14 Stockholm (SE)
(72) Inventor: ERIKSSON, JanOlof, 931 57 Skellefteå (SE)
(74) Representative: Swea IP Law AB
(86) International application number: PCT/SE2017/050525
(87) International publication number: WO 2017/200476

(56) References cited:
- WO-A1-2013/130969
- WO-A1-2015/038788
- Gunnar Sandström: "A new Product, BioCool , Tested on Acne Vulgaris. Can it provide a Positive Clinical Effect?", International Journal of Applied Science and Technology, vol. 6, no. 1 1 January 2016 (2016-01-01), 1 February 2016 (2016-02-01), pages 17-20, XP055389366, Retrieved from the Internet: URL:http://www.ijastnet.com/journals/Vol_6 _No_1_February_2016/3.pdf [retrieved on 2017-07-10]

## Description

### TECHNICAL FIELD

The present invention relates to a product for use in treatment of the skin for patients with Acne Vulgaris and/or Diabetic Peripheral Neuropathy.

### BACKGROUND ART

Acne vulgaris is a long-term chronic inflammatory skin disease in thousands of skin pores. It is a complex reaction chain with increased sebum production and abnormal keratinization in the pore. This leads to the growth of Propionibacterium acnes (P. acne) which triggers an immune reaction and inflammation in the skin pores, giving rise to lesions in the skin. (1, 2)

P. acne is an anaerobic gram-positive bacterium which prefer oxygen-poor environment, as is the case in skin pores. They are increasing in number and live on sebum produced by the sebaceous glands. P. acne itself is not harmful for the skin.

The diagnosis of acne vulgaris is often easy to set simply by looking at the follicular lesions, the age of the patient and the localization of the lesions (in the face and sometimes even on top of the chest, shoulders and back). Many of the patients are in a vulnerable teenage period. For these patients, acne causes a severe psychosocial stress correlated to acne difficulty (3). Acne usually improves around the age of 20, but may persist into adulthood. Permanent physical scarring may occur. There is also evidence to support the idea that acne has a negative psychological impact, and that it worsens mood, lowers self-esteem, and is associated with a higher risk of anxiety, depression, and suicidal thoughts. Another psychological complication of acne vulgaris is acne excoriée, which occurs when a person persistently picks and scratches pimples, irrespective of the severity of their acne. This can lead to significant scarring, changes in the affected person's skin pigmentation, and a cyclic worsening of the affected person's anxiety about their appearance.

There exist many different treatments for acne, including alpha hydroxy acid, anti-androgen medications, antibiotics, antiseborrheic medications, azelaic acid, benzoyl peroxide, hormonal treatments, keratolytic soaps, nicotinamide, retinoids, and salicylic acid. The main mechanisms for treatment are anti-inflammatory effects (benzoyl peroxide, retinoids, antibiotics, azelaic acid, salicylic acid, nicotinamide), hormonal manipulation, killing of P. acnes (benzoyl peroxide, antibiotics, azelaic acid), and /or normalizing skin cell shedding and sebum production in the pore to prevent blockage (retinoids, salicylic acid). Also light therapy, photodynamic therapy, is sometimes used to treat acne vulgaris.

Treatments that work in the long term and provide good clinical effect are difficult to find on the market. Long courses of oral antibiotic treatment of acne is the most common treatment and have been used for over 50 years (4). The treatment is usually 3-6 months and repeated in periods of deterioration. It involves treatment with tetracycline, which is a broad-spectrum antibiotic that presents risks of resistance development in the use of oral therapy. Consequently, the use of antibiotics may lead to an increasing development of resistance towards the antibiotic. It is therefore desirable to find alternative treatments to acne vulgaris, in order to prevent the risk of development of resistance. Furthermore, most treatments of acne vulgaris are topical, such as creams and ointments. WO 2005/018629 discloses a composition for treatment of acne vulgaris, comprising primarily etoxylate and sodium lauroyl sarcosinate. WO 2010/080543 discloses formulations to be used for treatment of acne vulgaris. The formulations are preferably applied as a cream, lotion of an emulsion. These formulations often have side effects and causes skin irritation (1). WO 2015038788 A1 discloses a composition for treating i.a. acne, comprising a chloride salt, a source of bicarbonate, a source of hypochlorite and an antimicrobial agent in the form of a pate, gel or ointment. JP 2009046457 A discloses a bathing agent or cleaning agent comprising a photocatalyst, apatite and an active agent, for the reduction of inflammation or improve acne. WO 2007099398 A2 discloses a composition for treatment of acne, comprising one or more metasilicate, one or more carbonate, one or more glyconate and one or more sulfate. Photodynamic treatment, which is sometimes used on severe acne vulgaris, causes severe redness and moderate to severe pain and burning sensation, and has also failed to show a statistically significant improvement (5). There is thus a need for effective treatments against acne vulgaris with fewer side effects

Diabetes Mellitus, both Type I (IDDM, Insulin-Dependent Diabetes Mellitus) and II (NIDDM, NonInsulin-Dependent Diabetes Mellitus), are common diseases worldwide, wherein a dis-balance between the demand and the production of the hormone Insulin occurs. In both cases, the consequence is that the patient will suffer from high blood sugar levels over a prolonged period, hyperglycaemia, causing the symptoms of a large amount of urine to be produced, increased thirst, and increased hunger. Over time hyperglycaemia damages nerves and blood vessels, leading to complications such as heart disease, stroke, kidney disease, blindness and dental disease.

Diabetic Neuropathy the most common complication associated with both IDDM and NIDDM, where nerve damage occurs. Relatively common conditions which may be associated with diabetic neuropathy include third nerve palsy; mononeuropathy; mononeuropathy multiplex; diabetic amyotrophy; a painful polyneuropathy; autonomic neuropathy; and thoracoabdominal neuropathy. High blood sugar causes injuries to nerve fibres throughout the body, but most often the nerve damage afflicts the legs and feet. Diabetic neuropathy affects all peripheral nerves including pain fibres, motor neurons and the autonomic nervous system.

Lower extremity complication in persons with diabetes is common due to peripheral neuropathy. The term Diabetic Foot is common within the field. Patients with Diabetic Peripheral Neuropathy and Diabetic Foot experience decreased sensation and loss of reflexes which first occurs in the toes on each foot, and then may extend upward. It is usually described as glove-stocking distribution of numbness, sensory loss, dysesthesia and night time pain. Pins and needles sensation is common. Loss of proprioception, the sense of where a limb is in space, is affected early. These patients cannot feel when they are stepping on a foreign body, such as a splinter, or when they are developing a callous from an ill-fitting shoe. Due to a loss of elasticity in the skin, caused by the neuropathy, the skin is more prone to crack and develop ulcers and lesions. Consequently, they are at risk of developing ulcers and infections on the feet and legs.

The development of a foot ulcer in individuals with diabetes mellitus and a diabetic foot usually involves several mechanisms, such as neuropathy, increased biomechanical stress and/or external trauma . The protective skin of the epidermis in individuals with diabetes and neuropathy is often impaired with dry skin and cracked skin caused by impaired epidermal lipid layers and increased transdermal water loss , and also loss of elasticity which increases the risk of foot ulcers and vulnerability for infection.

Approximately 5 % of the Scandinavian population is estimated to have diabetes mellitus. The problems of the lower extremity and development of foot ulcers are severe complications associated with an increased risk for a lower leg amputation. 50-85 % of lower leg amputations, excluding trauma/tumor, has been related to Diabetes mellitus. Up to 85% of diabetes related amputations are preceded by a foot ulcer of which 4 out of 5 are precipitated by a trauma to the vulnerable foot. A prevalence of 7-10% with regard to foot ulcers have been reported in the adult population with diabetes mellitus.

A strategy involving screening of high risk feet in individuals with diabetes mellitus, preventive foot /skin care, orthodic treatment and multidisciplinary treatment of established ulcers have been estimated to reduce diabetes related amputations with 62-81%. In that strategy of preventive foot care, an optimized skin condition is an important part to avoid or reduce risk of foot ulcers.

EP0773020 discloses the use of L-carnitin and alkanoyl L-carnitine for the prophylaxis and treatment of for instance Diabetic Peripheral Neuropathy. Current treatment standards against symptoms associated with Diabetic Peripheral Neuropathy and diabetic foot comprises foot care, a focus on diet, exercise, and correct foot wear. Also emollients may be used to soften the skin and facilitate foot care. Thus, there is at present time no effective treatment available for the treatment of Diabetic Peripheral Neuropathy and diabetic foot.

BioCool^{®} is classified as a medical device Type 1, and comprises sodium percarbonate (Na₂CO₃ · 1.5H₂O₂). The product is CE marked and approved by the FDA. It has in a previous study been demonstrated that the product kills microorganisms in a release of oxygen molecules (6). Furthermore, Saeed, A., et al., Vol. 5, No. 3, June 2015, IJAST, additionally shows that the product kills off microorganisms in Diabetic foot ulcers. The product has good clinical observed effect on nail fungus, athlete's foot and improves the symptoms of psoriasis.

WO2013130969 shows a topical wound treatment composition comprising a hydrogen peroxide generator; alkaline powder; not more than 5 percent by weight of water; additional topical active agent if desired, and emollient to balance, for topical application to a wound for treatment or healing thereof.

### SUMMARY OF THE INVENTION

The main objective of the present invention is to provide a composition that may ameliorate the skin condition for patients with Acne vulgaris or Diabetic Peripheral Neuropathy.

It is also an objective to provide a composition that may prevent the development of follicular lesions on the skin associated with Acne vulgaris. A further objective is to provide a composition that prevents the development of foot ulcers associated with Diabetic Peripheral Neuropathy.

In a first aspect, the present invention relates to composition comprising sodium percarbonate for use in the treatment of skin-related symptoms associated with indications chosen from Acne vulgaris or Diabetic Peripheral Neuropathy, wherein the treatment is topical, wherein the composition comprises 90-100 % of sodium percarbonate and the sodium percarbonate is provided as a dry formulation.

Furthermore, the present invention relates to the composition comprising sodium percarbonate above, for use in the treatment and prevention of follicular lesions associated with Acne vulgaris.

The present invention also relates to the composition comprising sodium percarbonate above, for use in the treatment and prevention of foot lesions associated with Diabetic Peripheral Neuropathy.

In a second aspect, the present invention relates to a kit-of-parts comprising a container with 10-20 tablets comprising 1g of sodium percarbonate, and a spray bottle capable of receiving 1 dl of liquid, for use in the topical treatment of Acne vulgaris.

### BRIEF DESCRIPTION OF THE DRAWINGS

Fig.1a: Figure showing the general mechanism involving H₂O₂ in professional phagocytes
Fig 1b: Figure showing the emission of reactive singlet oxygen molecules from hydrogen peroxide.

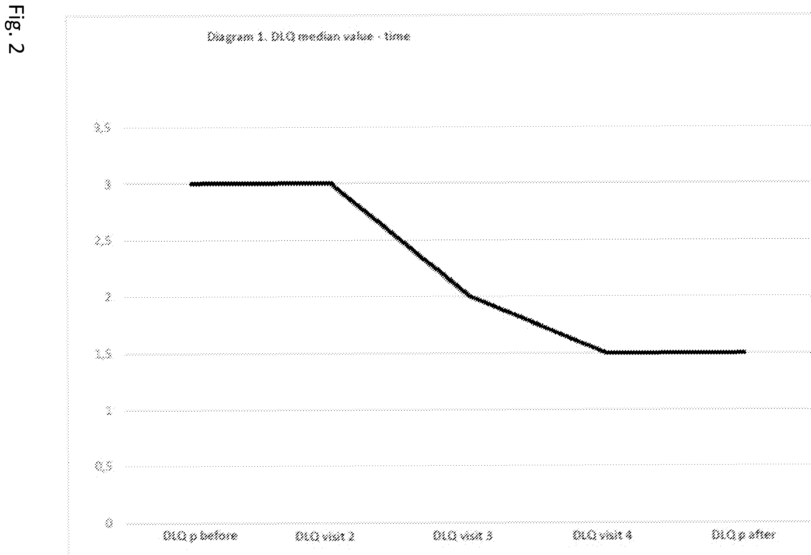

Fig.2: Diagram 1 showing the DLQ symptom rating, median value against time.

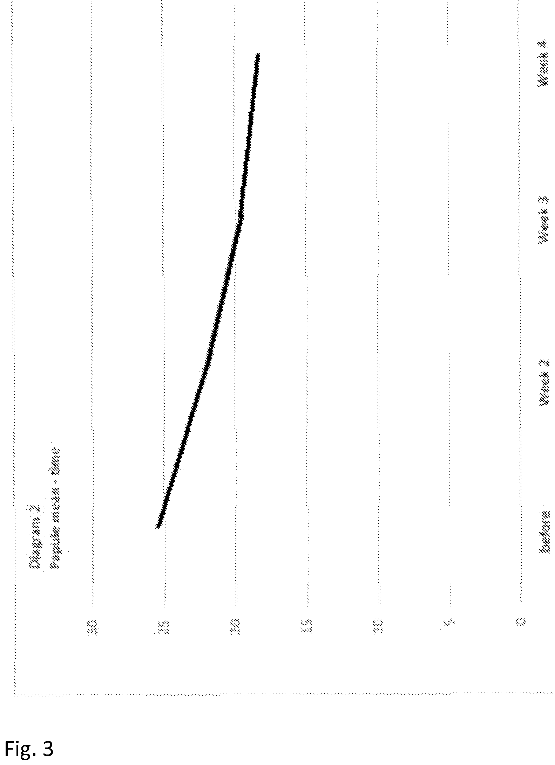

Fig. 3: Diagram 2 showing papule counting, mean value against time.
Fig. 4: Feet of one Diabetic Peripheral Neuropathy patient before (a) and after (b) treatment
Fig. 5: Feet of a second Diabetic Peripheral Neuropathy patient before (a -left foot, c -right foot) and after (b - left foot, d - right foot) treatment.
Fig 6: Feet of a third Diabetic Peripheral Neuropathy patient before (a) and after (b) treatment

### DETAILED DESCRIPTION

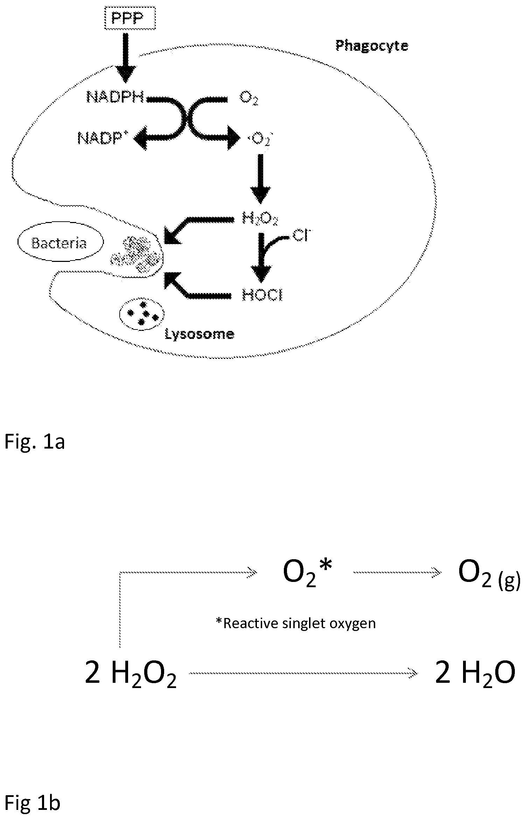

Professional phagocytes are a type of cells that protect the body by ingesting, and sometimes digesting, foreign particles such as bacteria or other microorganisms, as well as dust, dye but also other dead cells. Activation of professional phagocytic cells results in H₂O₂ production through the dismutation of O₂- generated by a membrane-bound NADPH oxidase (see Fig. 1a). One mechanism of action of the professional phagocytes is an oxygen-dependent production of superoxide, wherein superperoxide is converted to hydrogen peroxide and singlet oxygen molecules. When the oxygen molecules decomposes into water, these singlet oxygen molecules become effective against microorganisms by causing damage to the cellular membranes and inactivation of enzymes.

In addition to the antimicrobial effect of the singlet oxygen molecules, they also have a simulating effect on peripheral microcirculation. This has been proven for hydrogen peroxide at concentrations of 3%. However, such a concentration cannot be used daily due the risk of causing damage to the skin, such as irritation, allergic reactions, blisters, redness, skin burns, and ulcers.

The present invention relates to a composition comprising sodium percarbonate for use in the treatment of skin-related symptoms associated with indications chosen from Acne vulgaris and/or Diabetic Peripheral Neuropathy, wherein the treatment is topical.

Sodium percarbonate is a substance that upon dissolution in water will yield a mixture of hydrogen peroxide and sodium carbonate according to the following:

2Na₂CO₃.3H₂O₂ -> 2Na₂CO_{3(aq)} + 3H₂O_{2(aq)}

The hydrogen peroxide thereafter decomposes into water and oxygen:

2H₂O₂ → 2H₂O + O₂ (g)

This decomposition will generate singlet oxygen molecules, which are briefly available in the aqueous solution (see Fig. 1b). The time span wherein each singlet oxygen molecule is available in the aqueous solution is a few microseconds. However, the amount of singlet oxygen molecules at each given time is remarkable. By this mechanism, the sodium percarbonate may thus have the same effect on microorganisms as has been observed for professional phagocytes, with the additional effect of stimulating peripheral microcirculation.

However, in order to take advantage of the singlet oxygen molecules produced from the sodium percarbonate, it is not possible to formulate the composition in a cream, ointment or the like. In such a formulation, the hydrogen peroxide is stable by the addition of a stabilizing agent, and will not decompose and release the singlet oxygen molecules.

According to the present invention, the composition comprising sodium percarbonate is therefore provided as a dry formulation, such as in the form of granulate, a powder or beads. Preferably the dry formulation is chosen from granulate or powder. The dry formulation may furthermore be pressed into a tablet in order to facilitate dosage of the composition. 1 g of sodium percarbonate equals approximately 1 ml.

The composition may be used for the treatment of Acne vulgaris and for treatment and prevention of follicular lesions associated therewith.

When the composition is used for such a treatment, 1 g sodium percarbonate is dissolved in 1 dl of water at a temperature of 35-40 °C to form an active solution. Preferably the sodium percarbonate is in the form of a tablet when used to treat Acne vulgaris and follicular lesions associated therewith.

According to the present invention, a method for treatment of Acne vulgaris with the composition comprising sodium percarbonate, comprises the steps of:
- dissolving 1-5 g of sodium percarbonate in 0,05-1 dl of water at a temperature of 35-40 °C to obtain an active solution;
- application of the active solution onto the skin;
- leaving the solution on the skin for 5-10 minutes;
- washing the skin with water.

The solution obtained according to the method above is effective for about 24 h. The effectiveness of the solution is gradually decreased. A new solution must be prepared after 24 hours as the effectiveness of the singlet oxygen in the solution has decreased below an efficient level for treatment. The solution is to be applied to the face and/or affected skin at least once daily.

The solution will be prepared with different proportions depending on the form of application used. The solution may be sprayed onto the skin to be treated, or may be applied to the skin by hand, or by means of pads, cotton, cotton pads, a compress, tissue such as paper tissue. Also applying a compress in the form of patch impregnated with the solution is possible to use for the application. The solution may also be sprayed onto the pads, cotton, cotton pads, compress, and tissue. A solution that is intended to be sprayed is typically prepared by dissolving 1 g of sodium percarbonate in 1 dl of water.

A solution that is to be applied directly by hands, or directly by pads and the like according to the above, is typically prepared by dissolving 5ml sodium percarbonate in 5 ml of water, to achieve a smaller volume, and a more paste-like solution that is easierto handle. Such a solution may also be applied by the use of spatula.

The sodium percarbonate may also be dissolved or mixed with an aqueous gel. For instance water comprising a thickening agent such as for instance starch, vegetable gums or pectin may be used as the aqueous gel. 5 ml of sodium percarbonate may be added to 5 ml of the aqueous gel. Such a gel will be easy to handle and apply to the skin using the hands or fingers, but also here pads or the like according to the above, or a spatula may be used.

In the case where a solution of 1 g of sodium percarbonate and 1 dl of water is used, the solution will have a concentration of about 1% hydrogen peroxide that will decompose, causing a release of singlet oxygen molecules. As the water in the solution applied to the skin evaporates, the concentration of hydrogen peroxide on the skin will increase, until it is rinsed off with water during the final washing stage.

The effect of the treatment of Acne vulgaris is threefold: antimicrobial; stimulatory of peripheral circulation; and drying of the skin. The antimicrobial effect of the solution will kill off the P. acne present in and around the lesions. This leads to a decrease of the inflammation, papules, caused by P. acne in the pores. Additionally, other skin bacteria are killed off by the treatment. Furthermore, the treatment will dry out the skin, in reducing the sebum production, thus making the skin less suitable for further P. acne colonization. The stimulating effect of the peripheral microcirculation will increase the ability of the skin to heal the lesions, and increase the possibility for the immune system to prevent further bacteria to enter the pores. One side effect mentioned in connection with the Acne vulgaris treatment was redness and a slightly heating sensation. This is due to the increased circulation in the skin as a consequence of the treatment, which in fact is a positive effect. Inflammation in tissues in connection with the papules will be decreased, and any pus or exudes will also dry out, contributing to the healing of the skin and lesions. Thus, the composition for use according to the present invention has a positive clinical effect on Acne vulgaris, and in reducing papules and lesions associated with Acne vulgaris. Within 2 weeks of treatment, there is an apparent effect on the appearance of new papules, and the skin has started to heal and feels smoother.

The present invention also relate to a kit-of-parts comprising (1) a container with 10-20 tablets comprising 1g of sodium percarbonate, and (2) a spray bottle capable of receiving 1 dl of liquid, , for use in the topical treatment of acne vulgaris according to the above.

By adding 1 dl of water to the spray bottle, and then adding 1 tablet comprising 1g of sodium percarbonate, a solution is obtained in said bottle. Thereafter, the solution may be sprayed onto the face directly, or onto a compress, a pad, cotton, or the like, for subsequent padding on the face.

The composition according to the present invention is also for use in treatment of skin, more particularly on the feet, for patients with Diabetic Peripheral Neuropathy, and for treatment and prevention of foot lesions associated therewith. For this treatment, the composition is used in a foot bath.

The composition comprising sodium percarbonate in an amount of 15-60 ml is added to 2-3 L of water at a temperature of 36-38°C, preferably 37-38°C, and not exceeding 38.0°C. Preferably 40-60 ml of the composition is added to the water. Thus a solution is obtained comprising 0.16 -2 % hydrogen peroxide.

According to the present invention, a method for treatment of Diabetic Peripheral Neuropathy with the composition comprising sodium percarbonate, comprises the steps of:
- preparing a footbath of 2-3 L of water at a temperature of 36-38°C, not exceeding 38.0°C;
- placing the feet to be treated in the footbath;
- adding 15-60 ml, preferably 40-60 ml, of sodium percarbonate to the footbath;
- keep the feet in the bath for 15-25 minutes, preferably about 20 minutes;
- remove the feet from the bath;
- wipe feet dry.

The treatment during the foot bath will kill any microorganisms present on the skin, or in any lesions present, caused by the Diabetic Peripheral Neuropathy. In addition, the peripheral microcirculation will be stimulated, improving the ability of the skin to repair itself and heal the lesions. The loss of sensation in the foot will be reversed, and sensation will be regained. Furthermore, the foot bath will soften the skin, and facilitate removal of biofilms, such as a scab, from the lesions, thus making it possible for the foot bath solution to access and treat the inside of the lesions or wounds. The killing off of bacteria in the lesions will increase the wound healing capacity and decrease wound healing time. It has been found that although all bacteria are killed off in the treatment, any benign bacteria, usually present on the skin, will very quickly, within 24 hours, re-occupy the skin. However, malignant bacteria did not recur on the skin for days. Consequently, these effects in combination lead to a very favourable treatment for a condition where no current effective treatment currently exists.

The concentration of sodium percarbonate to be used in the foot bath will depend on the severity of the symptoms. Lighter symptoms may be treated with a lower concentration, such as 15-40 ml sodium percarbonate for 2-3 L of water. More severe symptoms will be treated with 40-60 ml sodium percarbonate. The treating physician is able to determine the severity of the symptoms as is customary for the person skilled in the art, and to determine a suitable dose to be used in the footbath.

The composition according to the present invention comprises 90-100 % of sodium percarbonate. It may further comprise a maximum of 5 - 10 % of other substances. These other substances are sodium chloride and sodium sulphate.

By the use according to the present invention, the hydrogen peroxide will not be stable in the aqueous solution, whereby it can act effectively on the skin in low concentrations while forming singlet oxygen molecules. The concentration of hydrogen peroxide formed in the aqueous solution is lower than the corresponding concentration of hydrogen peroxide in the liquid formulations that have been used historically. The concentration used historically is a solution of 3-5 % of hydrogen peroxide. Dissolving the sodium percarbonate in water according to the present invention will lead to a concentration of 0.16 - 2 % of hydrogen peroxide, which is highly effective, but significantly lower than the previously used formulations. It has been observed that within the range of 0.5-4 % of hydrogen peroxide, the effect on killing off microorganisms is practically the same although increasing concentration. Consequently, there is no significant gain in using the higher concentration in accordance with the traditionally used formulations. The present invention therefore aims at using lower concentrations of hydrogen peroxide to provide the effect. Thus the side effects associated with previously used formulations are decreased.

The present invention makes use of a well-known reaction between sodium percarbonate and water. Dissolving the sodium percarbonate in any other liquid than water, would lead to other reactions of the sodium percarbonate, and consequently another effect would be exerted. By dissolving the sodium percarbonate in water, the loss of oxygen is kept at a minimum. If any surfactants or other organic substances, solutions or material are added to the formulation, these will consume oxygen from the solution. This consumption of the oxygen in the solution will negatively impact the release of the singlet oxygen molecules, and consequently also negatively impact the treatment and the result. Thus, the sodium percarbonate is according to the use of the present invention, only to be dissolved in water.

The present invention will now be explained in examples. These examples are to further illustrate the invention and are not to be construed as limiting the scope of the invention, which is that of the appended claims

### Experiment 1 - Treatment of Acne Vulgaris

A study consisted of a total of 16 patients, 2 males and 14 females. Mean age was 28±7 years. The study included men and women, previously healthy, between 18-45 years, not using contraception, not breastfeeding or pregnant, and having been diagnosed with moderate acne. Patients with ongoing treatment for acne vulgaris were excluded or treatment was withdrawn one month before the start of the study. Also, patients who could not follow instructions and who were assumed to have poor compliance were excluded.

Statistical Methods: Dropouts included in the result were 2 patients. These patients were followed up at the doctor's appointment number 2 and the results were included in the so-called "intention to treat" where the values produced two visits was the same for the coming weeks for follow-up visits. The statistical analysis used was t-test and Wilcoxon rank-sum test.

Treatment and follow-up: On the first visit to a doctor, patients received the same information and instructions as given by the manufacturer BioCool^{®}. Each patient received the sodium percarbonate at home. The jars of powder were unmarked but if the patient wished to know the content they were informed. All processing was done at residences of the patient by the patient. 5 ml of sodium percarbonate was dissolved in 5 ml of water at a temperature of 35-40°C to form a solution. The solution was applied to the face using the fingers, and washed away with tap water after 5 minutes. This procedure was repeated daily by the patient. After about one hour after the treatment the subjects could, if desired, lubricate the skin with a moisturizing product, allowing enough time for the skin pores to close up for any additional product. Follow-up visits occurred weekly for four weeks. At each visit, the patient had completed the rating scale DLQ (Dermatology Life Quality Index), they were photographed and subject to an open interview.

Photography: Each patient was photographed prior to initiation of treatment, and weekly for four weeks. Portrait photo is taken from the front and profile of each view of the face. Photos were taken with the same camera and camera settings and the same photographer. The same light was used by 45-degree angle. However, technology is not validated and all the photos were not taken exactly the same. Each patient was encoded by the study manager with a number. After all the photos were taken, outside observers (fellow doctor) counted the number of acne papule for each patient for each visit, without knowing the visit week during which the photo was taken. The number of acne papules was summed up by frontal view, as well as by the two side views. Results were calculated as an average of the number of acne papule for each visit at the group level. DLQ survey summed with points for each patient for each visit. Even this was calculated with the average at group level.

DLQ (Dermatology Life Quality Index) is the first questionnaire designed specifically for skin diseases related to life and health. It is designed for people older than 18 years. The questionnaire is validated. It can be used for 40 different skin diseases. It contains 10 questions concerning symptoms and experience associated with skin disease. Acne disease affects quality of life and psychosocial well-being (3). Possible answers for symptoms / feeling are "very much", "very", "little", "nothing at all". These questions concern the past week. (7). Patients filled in a questionnaire at each visit.

Interview: The patient's subjective experience was investigated with a semi-structured interview based on the query field (8). The interview was conducted by research doctors. The text material was the question: "How do you perceive the treatment?" Follow-up question could be "Describe your feeling in the skin," "Can you give examples of the effect on the skin." After each follow-up visit the patient's response was noted directly in the patient record. No recording method was used.

The texts were compiled and key words were taken out, for example, dryness, itching, burning, and the theme around the treatment procedure was time-consuming and messy.

### Results

In the present study, 87.5% of subjects are women and the average age estimated to 28 years. Values for calculating acne papule was normally distributed and the average before starting treatment was 25.4 and after 4 weeks 18.3 i.e. a reduction and improvement in the number acne papule. The result of the number of papule reduced over time was significant with p-value <0.001.

**Table 1. Background data and results for DLQ symptom rating and papule counting for 4 weeks of treatment with Sodium percarbonate. Mean, median or percentage plus Standard Deviation or interquartile range as well as p value are given.**

| Table 1. Background data and results for DLQ symptom rating and papule counting for 4 weeks of treatment with Sodium percarbonate. | | | |
|---|---|---|---|
| | Mean, median^{∗} or percentage | Standard Deviation or interquartile range^{∗} | p value |
| Age | 28 | 7.4 | |
| Sex (% female) | 87.5 | | |
| DLQ: | | | |
| Before ^{∗} | 3 | 4 | |
| After 4 weeks^{∗} | 1.5 | 2 | 0.002 |
| Papule count: | | | |
| Before | 25.4 | 12.4 | |
| After 4 weeks | 18.3 | 10.2 | <0.001 |

The DLQ values were not normally distributed and the median before the initiation of treatment was estimated at 3 and then after 4 weeks of treatment to 1.5. The interquartile range (IQR) was 4 and 2, respectively, with significant p-value of 0.002.

The number of acne papule declined over time and additionally patients estimated fewer points on the DLQ.

Dropouts: Two patients discontinued treatment after two weeks. One patient had more redness and itching on the skin which is why treatment was topped. The second patient was traveling and could not complete the study. The loss was included in the result. These patients were followed up at the doctor's appointment number 2 and the results were included in the so-called "intention to treat".

During the patient interviews it was described that the patients' skin felt cleaner, smoother, softer and firmer. Acne papule did not break out as often as before. They felt that they received less signs and symptoms of acne. Some drawbacks were also mentioned by some subjects, such as that the treatment was perceived as messy, the procedure was perceived as time-consuming and it was difficult to plan the timing of the procedure as the skin could not be moisturized until an hour after the treatment. Also, in some cases, the skin felt very dry after application. (Table 2).

**Table 2. Interview. Summarizes patient's total experience for the four-week period of treatment with Sodium percarbonate.**

| Patient no | Key word | Experience of treatment |
|---|---|---|
| 1 | Dry | Time consuming |
| 2 | Smooth skin | Good |
| 3 | Burning | Messy |
| 4 | Dry | Messy |
| 5 | Smooth skin | Good |
| 6 | Smooth skin | Good |
| 7 | Smooth skin | Good |
| 8 | Smooth skin | Good |
| 9 | Dry | Messy |
| 10 | Smooth skin | Messy |
| 11 | Smooth skin | Messy |
| 12 | Smooth skin | Messy |
| 13 | Dry | Good |
| 14 | Smooth skin | Good |
| 15 | Dry | Messy |
| 16 | Dry | Irritation |

### Discussion

The number of acne papule was clearly reduced and patients estimated fewer symptoms for each week under the DLQ. The most common side effect was dry skin according to the interview questions.

In one study the effectiveness was analyzed and safety of different local agents (gel) for acne vulgaris and the number of acne papule was counted. The results showed that benzoyl peroxide 2.5 % and clindamycin 1 % was effective (number acne papule reduction) and the combination of benzoyl peroxide and nadifloxacin 1 % showed better safety profile, which is statistically significant. In a third group tretinoin 0.025 % and clindamycin 1 % were analyzed. They found a statistically significant reduction in the number of acne papules in those treated with benzoyl peroxide and clindamycin. (9)

In a randomized study the efficacy, safety and tolerability were analyzed of Adapalene and benzoyl peroxide in patients with mild acne vulgaris. In both groups the number acne papules were significant reduced. Both treatments appear to be safe and effective in mild acne vulgaris. (10).

There are similarities in this pilot study and the aforementioned studies, which also have significant results in reducing the number of acne papules.

Sodium percarbonate releases hydrogen peroxide which in turn decomposes to free oxygen molecules that kill anaerobic bacteria (11). In a randomized study it was looked at the difference in treatment between benzoyl peroxide and hydrogen peroxide cream to moderate acne vulgaris. The result showed that the hydrogen peroxide is at least as good and also better tolerance for skin (12).

Sodium percarbonate has great development potential. Patients were very satisfied with the continuous medical attention and appreciate the expertise. They gained better insight into their skin and developed better hygiene routines. Also the number of papule significantly decreased.

Using open questions, patients have been telling about the experience, both negative and positive effects. Some common keywords have been verified, which is strength. Dry skin was the most common side effect of the treatment. However, since acne is associated with an overproduction of sebum, this drying out effect of the treatment is unfavorable for acne. Hence, this side effect can be seen as a positive effect (13). One patient discontinued the study due to wrong diagnosis earlier. It is usual to be mistaken for acne rosacea (14). Photographing is performed with a non-standard approach, not validated, however, blinded which is a strength. DLQ survey has been used in previous research and validated (15).

### Experiment 2 - Treatment of Diabetic Peripheral Neuropathy

### STUDY OBJECTIVES

MAIN OBJECTIVE: To demonstrate, during a 6-week of evaluation with a 4 week follow-up, improved skin condition of the foot in individuals with moderate/severe skin lesions in individuals with diabetes mellitus

MAIN CRITERION OF EVALUATION: A favourable outcome (FHO) is the main efficacy criterion.

A FHO is considered to have occurred in a given patient if all the following endpoints are met:
I. Improvement in skin condition evaluated by photos according to a pre-set scale (1-5):

| **Does the patient have any of these conditions on the feet?** | | | | | | | |
|---|---|---|---|---|---|---|---|
| **Mark Yes/No and grade the severity.** | | | | | | | |
| | | | | | | | |
| | Yes | No | 1 | 2 | 3 | 4 | 5 |
| Eczema | | | Insignificant | Low | Moderate | Uttalad | Severe |
| | | | | | | | |
| Psoriasis | | | Insignificant | Low | Moderate | Pronounced | Severe |
| | | | | | | | |
| Itching | | | Insignificant | Low | Moderate | Pronounced | Severe |
| | | | | | | | |
| Athlete's foot | | Insignificant | Low | Moderate | Pronounced | Severe | |
| | | | | | | | |
| Nail fungus | | Insignificant | Low | Moderate | Pronounced | Severe | |
| | | | | | | | |
| Skin cracking | | Insignificant | Low | Moderate | Pronounced | Severe | |
| | | | | | | | |
| Dry feet | | Insignificant | Low | Moderate | Pronounced | Severe | |
| | | | | | | | |
| Calluses | | Insignificant | Low | Moderate | Pronounced | Severe | |
| | | | | | | | |
| Loss of sensation | | Insignificant | Low | Moderate | Pronounced | Severe | |
| | | | | | | | |
| Other: | | | | | | | |
| | | | | | | | |

| **How would you summarize the patients overall condition on the skin of the foot?** | | | | | | | |
|---|---|---|---|---|---|---|---|
| **Choose the alternative best suited.** | | | | | | | |
| | | 1 | 2 | 3 | 4 | 5 | |
| | | Insignificant | Low | Moderate | Pronounced | Severe | |
| | | | | | | | |

II. Per Protocol Improvement of skin condition according to above mentioned scale 1-5
III. Improvement of symptoms and signs of skin condition according to pre-set protocol by the user (patient)at home
IV. Improvement in skin condition and performance based on per protocol evaluation by podiatrist according to pre-set protocol

### SECONDARY OBJECTIVES:

### 5.3.1 Efficacy evaluations

1. Photos for independent evaluation.
2. Per Protocol regarding general skin condition according to the same scale as photo evaluation (grade 1-5)
3. Test protocol by chiropodist/podiatrist describing skin condition in detail (dryness, cracked skin, callosities, and superficial skin lesions)
4. Test protocol for patients for their evaluation of the treatment.

Three questionnaires are used:
- Two questionnaires for the patients. The first is completed before the patient starts the treatment and the other one after they finished treatment.
- One questionnaire to use before treatment starts and after finished treatment.

### 5.3.2 Safety evaluations

Description and prevalence of local and general adverse events (expected and unexpected adverse events.

### STUDIED POPULATION

### TARGET POPULATION

The target population include in this evaluation are adult individuals with diabetes and neuropathy, with feet with moderate to severe skin conditions

### INCLUSION CRITERIA

1. Written consent to participate obtained.
2. Male or female patient aged at least 18 years without superior limit.
3. Type 1 or 2 diabetes.
4. Sensory neuropathy evaluated by biotensiometer (vpt>25) or monofilament (Semmes - Weinstein 5.07) monofilament according to diagnosis criteria as is common practice within the field.
5. Skin lesions/skin condition evaluated as moderate or severe with regard to dryness, cracks, superficial skin lesions and callosities.
6 For both legs: ABPI (Ankle brachial pressure index) ≥0.6 and <1.3 AND ankle systolic pressure ≥70 mmHg or systolic toe pressure ≥50 mmHg or TcPo2 (Transcutaneous oxygen pressure) ≥30 mmHg (decubitus).
7. Patient compliant to and in medical and emotional condition to fulfil the study

### NON-INCLUSION CRITERIA

Patients meeting at least one of the following criteria are not included:
1. Previous history of major amputation or planned major amputation within the first month after randomization.
2. Revascularization procedure of lower leg, or any other local of any leg <4 weeks before inclusion.
3. Patient unable to be treated with Sodium percarbonate due to other concomitant therapy.
4. Presence of any ulcer (full skin ulcer Wagner grade1-5).
5. Any evidence of skin cancer, vasculitis or inflammatory skin condition.
6. Signs of skin infection in the lower leg.
7. Patient with a severe comorbid disorder or concurrent disease, that inhibits participation and capacity to follow the study protocol.
8. Patient who are in the exclusion period following a previous participation to another trial or who are currently participating to any concomitant study with any drug or device.
9. Known intolerance to the tested medical device .

### 7.STUDIED DEVICE

7.1 Sodium percarbonate, granulate, 750 g in a can.

7.1.1 Product description: Granulate that consists of an oxygen producing substance, H202.

7.1.2 Precautions. Do not exceed 38°C due to risk of burn of skin. Dust from granules powder may cause irritation to your eyes.

7.1.3 Frequency of application. Daily application for 6 weeks or until the signs and symptoms are cleared, then once a week.

7.1.4 Directions for use
1. Start by placing the feet in the footbath, containing 2-3 litres (about 1 gallon) of lukewarm water (about 38 °C). Measure out 15 ml (about 1 tbs.) of granules and add to the footbath. NB! The order is important, the feet must be in the bath before adding the granules.
2. Bathe the feet for about 20 minutes
3. Wipe the feet dry using a towel.

7.1.5 Storage: The packaging shall be stored in room temperature and well-sealed.

### 8 BEST LOCAL CARES

NONE AUTHORIZED DEVICES OR DRUGS DURING STUDY COURSE: No antifungal cream/ointment, no foot cream.

### 9.GENERAL DESIGN

### 9.1 GENERAL DESIGN

This is a 6-week, single centre, open label evaluation conducted in patients suffering from Diabetic Feet of neuropathic origin with moderate to severe non ulcer pathology (dry skin ,cracks , callosities)

### 9.2 STUDY FLOW CHART visits 0 and 6 weeks for photos, skin evaluation , test protocol and evaluation of safety and concordance

Laboratory measurements (Hemoglobin, Creatinine, HbA1c) within 6 weeks of inclusion

### 10 CONDUCT OF THE EVALUATION

### 10.1 PATIENT RECRUITMENT AND GENERAL CONDUCT OF THE EVALUATION

Screened patients are subjects meeting the selection criteria to whom investigator will propose to participate to the evaluation. They receive written information about protocol and study constraints and potential benefits and risks. They should have enough time to read this information and to ask any further questions to investigator. Then they will have to sign the consent form to be included.

Investigators has to use the CRF for the randomization after checking the selection criteria.

As soon as they are included, main patient medical history are recorded and all ulcers present at that visit will be described. Patients will be instructed on the use of Sodium percarbonate and patient test protocol

A wound photograph is performed for both feet (plantar surface or both). Local cares will be applied and the first application of the treatment will be done. Patients will receive a patient care diary. This diary has to be completed by the individual (patient) for every local cares performed during study course (at home)

### 10.1.1 Medical visits at inclusion and week 6

At each visit a required medical evaluations is performed at inclusion (0) and 6 weeks (+/-3 days). A photograph is performed and a test protocol is followed regarding skin condition. Any local and general intercurrent events (expected or unexpected) are recorded. Patients receive a patient care diary at their first visit. Investigators are allowed to see patients more frequently if required..

If during evaluation course, the Sodium percarbonate treatment has to be stopped for various reasons it will be recorded. If subject has to be dropped from the trial, a form will be completed including evaluation along with test protocol and photograph

### 10.2 DROPUOUTS DURING THE 6-WEEK STUDY PERIOD

The evaluation will primarily be evaluated according intention to treat (ITT). A per protocol evaluation will also be performed. All drop outs and withdrawals will be recorded and included in an ITTP analysis.

### 10.3 DATA RECORDINGS

### 10.3.1 Demographic data and history of the skin disease /condition

Sex and age.
Skin photo type of the lesions according to test protocol
Check list of inclusion and non-inclusion criteria.
Known duration of diabetes and type of diabetes (1 or 2).
List of diabetes complications other than peripheral neuropathy
Last HbAlc values (and date of exam).
Current listing of antidiabetic treatments.
History of previous foot ulcers
History of previous minor amputation and type of amputation.
Grading of skin lesions

Arterial blood pressure measurements, ABPI recording of both lower limbs and/or of ankle systolic pressure, systolic toe pressure and of TcPO2 (decubitus).
10.3.2 Clinical efficacy data
10.3.2.1 Evaluation of general skin condition. Grade 1-5
10.3.2.2 Evaluation of specific skin condition (dry skin, cracks, callosities)
10.3.2.3 Foot/Skin photography
10.3.3 Clinical safety data
10.3.3.1 Infection occurrence
10.3.3.2 Ulcer
10.3.3.3 Other safety parameters
10.3.4 Patient adherence to treatment and study protocol
10.3.4.1 Patient care diary - Questionnaire and Diary
10.3.4.2 Other general treatments

### 11 DATA HANDLING AND STATISTICAL ANALYSIS

11.1 SAMPLE SIZE CALCULATION : assumed 20 patients for initial evaluation.
11.2 RANDOMIZATION : not applicable
11.3 EVALUATION CRITERIA Improvement of skin condition evaluated by photos analysed by independent observers
11.4 STATISTICAL ANALYSIS PLAN
11.4.1 Principles
11.4.2 Calculated parameters Scales evaluating skin condition (grade 1-5)
11.4.3 Main study endpoint analysis. Improvement in general skin condition according to grade scale prior to intervention and after 6 weeks
11.4.4 Secondary endpoints. Changes in various skin conditions (dryness, cracked skin, callosities) performed per protocol by chiropodis
11.4.5 Other evaluations patient personal evaluation per protocol
11.4.6 Safety analysis all adverse reactions will be recorded
11.4.7 Interim analysis: not applicable
11.4.8 Statistical analysis plan: I, Mann WhitneyTT Chi2

### 12 MANAGEMENT OF ADVERSE EVENTS

12.1 LOCAL ADVERSE EVENTS Will be reported
12.2 INTERCURRENT AND GENERAL ADVERSE EVENTS is reported
12.3 ADVERSE DEVICE EFFECTS AND DEVICE DEFICIENCY is reported
12.4 SERIOUS ADVERSE EVENTS is reported

13 DATA HANDLING AND RECORD KEEPING Data is handled and recorded by Skåne Universitetssjukhus, address: Waldenströms gata 15 Endokrinologiska kliniken Skåne Universitetssjukhus Malmö 205 02 Malmö.

13.1 CONFIDENTIALITY. Data will be delivered without identification of individual participants. Participants will be given a participation number. A list will be prepared connecting individuals with those numbers. That list will be stored by the investigating centre

### PRELIMINARY RESULTS

Following treatment according to the study protocol above, a significant visual improvement of the condition of the feet is observed for the patients included in the study.

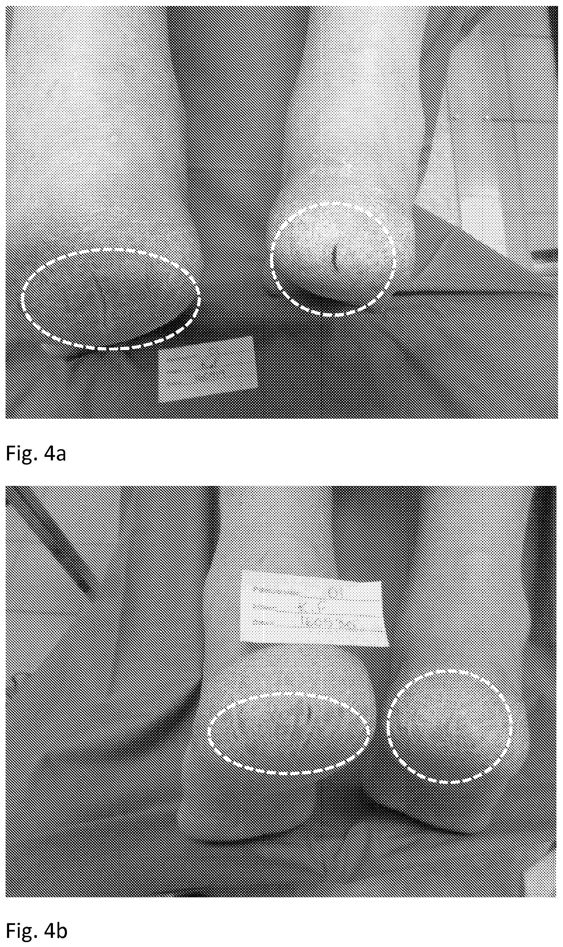

Fig. 4 shows the feet of patient No. 01 included in the study. In Fig. 4a, there are significant cracks in the skin on the heels of both feet. There is one large and deep crack on the right heel (marked with circle), and several moderate cracks on the left heel (marked with circle). Fig. 4b is a photo of the same patient after 6 weeks of treatment according to the study protocol. The large crack on the right heel has significantly improved, and on the left heel, most of the cracks have disappeared. An observed decline of dry skin can also be observed, as both heels after treatment have less white dry skin compared to the more prevalent presence of dry, white skin before treatment.

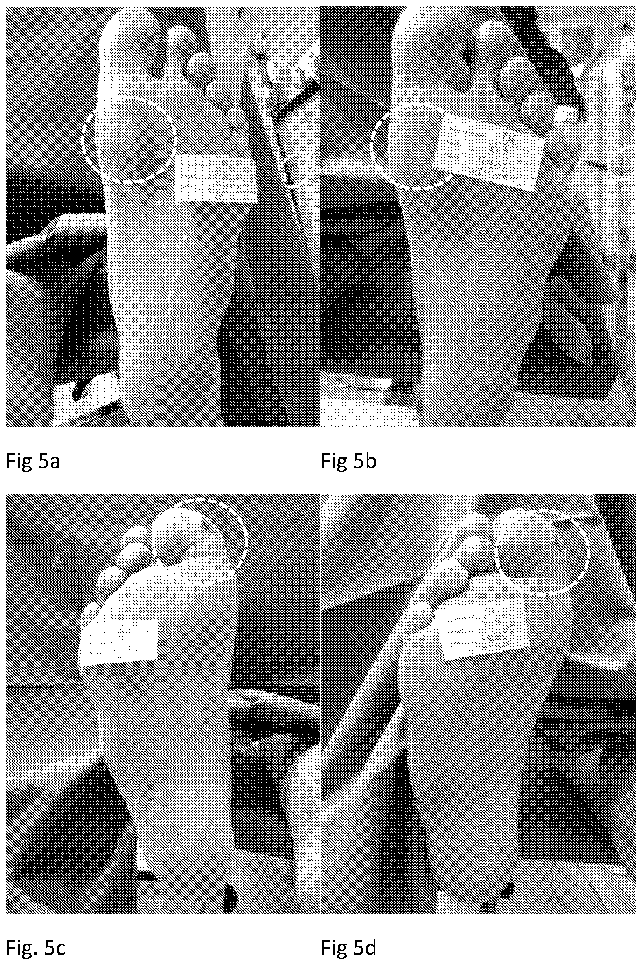

Fig. 5 a-d shows the feet of patient No. 06 included in the study. Figs 5a and 5b shows the left foot before (a) and after (b) treatment. Figs. 5c and 5d shows the right foot before (c) and after (d) treatment. Before treatment, both feet have dry white skin all over the soles, which is clearly diminished after treatment. On the left foot (5a) a hardened skin portion can be seen to the left on the footpad (marked with circle), which is no longer clearly visible on Fig. 5b. The right foot has a large ulcer on the big toe with dry skin surrounding it on Fig. 5c (marked with circle). In Fig. 5d, the ulcer has declined in size, and there is nearly no dry skin surrounding the ulcer.

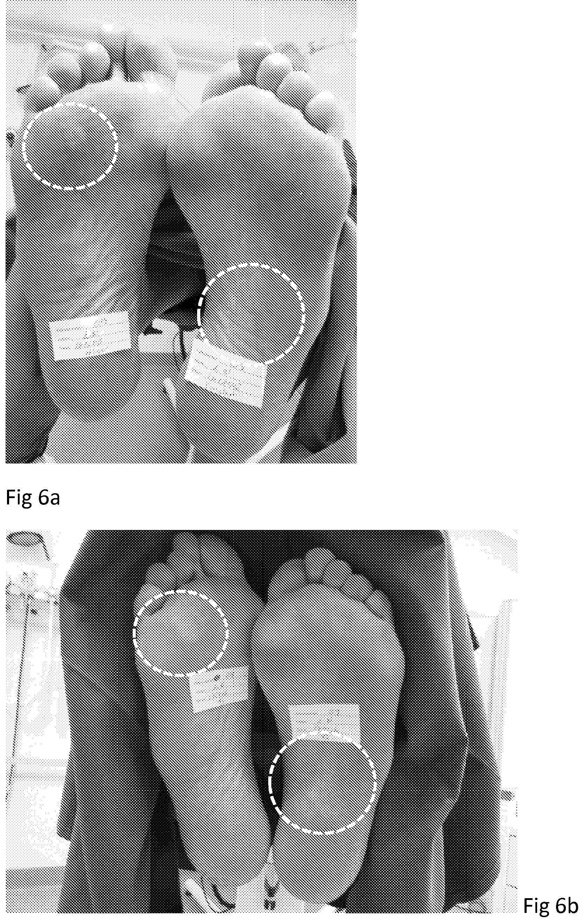

Fig. 6 shows the feet of patient No. 9 included in the study. Fig 6a is before treatment and shows several dry and hardened patches of skin visible on the footpad, in particular on the right foot (marked with circle). In fig. 6b, the skin has a healthier color and less of dry skin and the hardened patches are no longer present. An improvement of skin elasticity can also be observed, in particular for the left foot, where the skin in the foot arch (marked with circle) is very wrinkled in Fig 6a, but almost entirely smooth in Fig. 6b.

These results indicate a significant improvement of the skin condition in the Diabetic Peripheral Neuropathy patients following treatment with sodium percarbonate according to the study protocol. The improvements are visible relating to skin cracks, skin ulcers, dry skin and skin elasticity.

### References

1. Shannon Humphrey, MD, FRCPC, FAAD, Skin Therapy Letter. 2012; 17
2. Thiboutot D, Gollnick H, Bettoli V, et al. New insights into the management of acne: an update from the Global Alliance to Improve Outcomes in Acne Group. J Am Acad Dermatol. 2009; 60: S1-S50.
3. Halvorsen JA, Stern RS, Dalgard F Thoresen M, Bjertness E, Lien L. Suicidal ideation, mental health problems, and social impairment are Increased in adolescents with acne: a population-based study. J Invest Dermatol. 2011; 131: 363-70.
4. Dreno B, Bettoli V, Ochsendorf F, Layton A, Mobacken H, Degreef H. European Recommendations on the use of oral antibiotics for acne. Eur J Dermatol. 2004; 14: 391-9.
5 "DUSA Pharmaceuticals (DUSA) to Stop Developing Phase 2 Acne Treatment". Biospace. 2008-10-23. Retrieved 2009-07-30.
6. Gunnar Sandström, J-O Eriksson and Amir Saeed. Successful Treatment of Onychomycosis Trichophytonrubrum and Warts (Verruca Plantae) with BioCool®. International Journal of Applied Science and Technology Vol. No. 4 1; January 2014
7. Zachariae R, Zachariae C, Ibsen H, Mortensen JT, Wulf HC. Dermatology Life Quality Index: Data from Danish inpatients and outpatients.
8. Robert K. Merton and Patricia L. Kendall (1945/46) article, The Focused Interview
9. Kaur J, Sehgal HC, Gupta AK, Singh SP. A comparative study to evaluate the efficacy and safety of combination topical preparations in acne vulgaris. 2015 May-Aug; 5 (2): 106-10. doi: 10.4103 / 2229-516X.157155
10. Babaeinejad SH, Fouladi RF. The efficacy, safety, and tolerability of adapalene versus benzoyl peroxide in the treatment of mild acne vulgaris: a randomized trial. J Drugs Dermatol. 2013 Jul 1; 12 (7): 790-4.
11. Muizzudin N, Schnittger, Maher W, Maes, DH, T. Mammone enzymatically generated hygrogen peroxide Reduces the number of acne lesions in acne vulgaris. 2013 January-February; 64 (1): 1-8
12. Massimo Milani, Andrea Bigardi, Marco Zavattarelli. Efficacy and Safety of stabilized Hydrogen Peroxide Cream (Crystacide) in Mild-to-Moderate Acne Vulgaris: A Randomised Controlled Trial Versus Benzoyl Peroxide Gel. Curr Med Res Opin. 2003, 19 (2)
13. Bruggemann, et al. 2004. The Complete genome sequence of Propionibacterium acnes, a commensal of human skin
14. Is it Acne Rosacea or Is it? An Important Distinction. Cutis. 2012; 90: 59-61
15. Lekakh O, Mahoney AM, Novice K, Kamalpour J, Sadeghian A, Mondo D, Kalnicky C, Guo R, Peterson A, Heavy R. Treatment of Acne Vulgaris With Salicylic Acid Chemical Peel and Pulsed Dye Laser: A Split Face, Rater-Blinded, Randomized Controlled Trial. J Lasers Med Sci. Fall 2015; 6 (4): 167-70. doi: 10.15171 / jlms.2015.13. Epub 2015 Oct 27

## Claims

1. A composition comprising sodium percarbonate for use in the treatment of skin-related symptoms associated with indications chosen from Acne vulgaris or Diabetic Peripheral Neuropathy, wherein the treatment is topical, wherein the composition comprises 90-100 % of sodium percarbonate and the sodium percarbonate is provided as a dry formulation.

2. The composition for use according to claim 1, for treatment and prevention of follicular lesions associated with Acne vulgaris.

3. The composition for use according to any of claims 1 or 2, wherein 1-5 g sodium percarbonate is dissolved in 0.05-1 dl of water at a temperature of 35-40 °C to form an active solution to be applied to the skin by hand, by spray, or by means of pads, cotton, cotton pads, tissue such as paper tissue or by spatula.

4. The composition for use according to claim 1, for treatment and prevention of foot lesions associated with Diabetic Peripheral Neuropathy.

5. The composition for use according to any of claims 1 or 4, wherein the product is used in a foot bath.

6. The composition for use according to any of claims 1 or 4-5, wherein 15-60 ml of the composition is added to 2-3 L of water at a temperature of 36-38°C, preferably 37-38°C, and not exceeding 38.0°C.

7. The composition for use according to any of claims 1 or 4-6, wherein 40-60 ml of the composition, is added to 2-3 L of water at a temperature of 36-38°C, preferably 37-38°C, and not exceeding 38.0°C.

8. The composition for use according to any of the preceding claims, wherein the composition additionally comprises a maximum of 5-10 % of other substances.

9. The composition for use according to claim 9, wherein the other substances are sodium chloride and sodium sulphate.

10. The composition for use according to any of the preceding claims, wherein the sodium percarbonate is provided in the form of a granulate, a powder or beads.

11. The composition for use according to any of the preceding claims, wherein the dry formulation is pressed into a tablet.

12. A kit-of-parts comprising (1) a container with 10-20 tablets comprising 1g of sodium percarbonate, and (2) a spray bottle capable of receiving 1 dl of liquid, for use in the topical treatment of acne vulgaris according to any of the claims 1-3 or 8-11.

## Patentansprüche

1. Zusammensetzung, umfassend Natriumpercarbonat zur Verwendung bei der Behandlung von hautbezogenen Symptomen, die mit Indikationen verbunden sind, die aus Acne vulgaris oder diabetischer peripherer Neuropathie ausgewählt sind, wobei die Behandlung topisch ist, wobei die Zusammensetzung zu 90 bis 100 % Natriumpercarbonat umfasst und das Natriumpercarbonat als eine Trockenformulierung bereitgestellt wird.

2. Zusammensetzung zur Verwendung nach Anspruch 1 für die Behandlung und Vorbeugung von follikulären Läsionen, die mit Acne vulgaris verbunden sind.

3. Zusammensetzung zur Verwendung nach einem der Ansprüche 1 oder 2, wobei 1 bis 5 g Natriumpercarbonat in 0,05 bis 1 dl Wasser bei einer Temperatur von 35 bis 40 °C gelöst wird, um eine aktive Lösung auszubilden, die von Hand, durch ein Spray oder mittels Pads, Watte, Wattepads, Tüchern, wie Papiertaschentüchern, oder durch einen Spatel auf die Haut aufgebracht werden soll.

4. Zusammensetzung zur Verwendung nach Anspruch 1 für die Behandlung und Vorbeugung von Fußläsionen, die mit diabetischer peripherer Neuropathie verbunden sind.

5. Zusammensetzung zur Verwendung nach einem der Ansprüche 1 oder 4, wobei das Produkt in einem Fußbad verwendet wird.

6. Zusammensetzung zur Verwendung nach einem der Ansprüche 1 oder 4 bis 5, wobei 15 bis 60 ml der Zusammensetzung zu 2 bis 3 I Wasser bei einer Temperatur von 36 bis 38 °C, vorzugsweise 37 bis 38 °C, und nicht über 38,0 °C, zugegeben wird.

7. Zusammensetzung zur Verwendung nach einem der Ansprüche 1 oder 4 bis 6, wobei 40 bis 60 ml der Zusammensetzung zu 2 bis 3 I Wasser bei einer Temperatur von 36 bis 38 °C, vorzugsweise 37 bis 38 °C, und nicht über 38,0 °C, zugegeben wird.

8. Zusammensetzung zur Verwendung nach einem der vorstehenden Ansprüche, wobei die Zusammensetzung zusätzlich maximal zu 5 bis 10 % andere Substanzen umfasst.

9. Zusammensetzung zur Verwendung nach Anspruch 9, wobei die anderen Substanzen Natriumchlorid und Natriumsulfat sind.

10. Zusammensetzung zur Verwendung nach einem der vorstehenden Ansprüche, wobei das Natriumpercarbonat in der Form eines Granulats, eines Pulvers oder von Kügelchen bereitgestellt wird.

11. Zusammensetzung zur Verwendung nach einem der vorstehenden Ansprüche, wobei die trockene Formulierung in eine Tablette gepresst wird.

12. Teilesatz, umfassend (1) einen Behälter mit 10 bis 20 Tabletten, umfassend 1 g Natriumpercarbonat, und (2) eine Sprühflasche, die in der Lage ist, 1 dl Flüssigkeit aufzunehmen, zur Verwendung bei der topischen Behandlung von Acne vulgaris nach einem der Ansprüche 1 bis 3 oder 8 bis 11.

## Revendications

1. Composition comprenant du percarbonate de sodium pour une utilisation dans le traitement de symptômes liés à la peau associés à des indications choisies parmi l'acné commune ou la neuropathie périphérique diabétique, dans laquelle le traitement est topique, dans laquelle la composition comprend 90 à 100 % de percarbonate de sodium et le percarbonate de sodium est fourni sous la forme d'une formulation sèche.

2. Composition pour une utilisation selon la revendication 1, pour le traitement et la prévention des lésions folliculaires associées à l'acné commune.

3. Composition pour une utilisation selon l'une quelconque des revendications 1 ou 2, dans laquelle 1 à 5 g de percarbonate de sodium sont dissous dans 0,05 à 1 dl d'eau à une température de 35 à 40 °C pour former une solution active à appliquer sur la peau à la main, par pulvérisation, ou au moyen de tampons, de coton, de tampons de coton, de tissu tel que du papier-mouchoir ou à la spatule.

4. Composition pour une utilisation selon la revendication 1, pour un traitement et une prévention des lésions du pied associées à la neuropathie périphérique diabétique.

5. Composition pour une utilisation selon l'une quelconque des revendications 1 ou 4, dans laquelle le produit est utilisé dans un bain de pied.

6. Composition pour une utilisation selon l'une quelconque des revendications 1 ou 4 à 5, dans laquelle 15 à 60 ml de la composition sont ajoutés à 2 à 3 I d'eau à une température de 36 à 38 °C, de préférence 37 à 38 °C, et ne dépassant pas 38,0 °C.

7. Composition pour une utilisation selon l'une quelconque des revendications 1 ou 4 à 6, dans laquelle 40 à 60 ml de la composition sont ajoutés à 2 à 3 I d'eau à une température de 36 à 38 °C, de préférence 37 à 38 °C, et ne dépassant pas 38,0 °C.

8. Composition pour une utilisation selon l'une quelconque des revendications précédentes, dans laquelle la composition comprend en outre un maximum de 5 à 10 % d'autres substances.

9. Composition pour une utilisation selon la revendication 9, dans laquelle les autres substances sont du chlorure de sodium et du sulfate de sodium.

10. Composition pour une utilisation selon l'une quelconque des revendications précédentes, dans laquelle le percarbonate de sodium est fourni sous la forme d'un granulé, d'une poudre ou de billes.

11. Composition pour une utilisation selon l'une quelconque des revendications précédentes, dans laquelle la formulation sèche est pressée sous forme de comprimé.

12. Kit de pièces comprenant (1) un récipient avec 10 à 20 comprimés comprenant 1 g de percarbonate de sodium, et (2) un flacon pulvérisateur capable de recevoir 1 dl de liquide, pour une utilisation dans le traitement topique de l'acné commune selon l'une quelconque des revendications 1 à 3 ou 8 à 11.
